# EUROPEAN PATENT APPLICATION

(11) **EP 1 211 514 A1**
(43) Date of publication of application: **05.06.2002**
(21) Application number: 00956844.5
(22) Date of filing: 31.08.2000
(51) Int. Cl.: G01N 33/566, G01N 33/532, C12N 15/09, C07K 17/00, C07K 1/13, C12Q 1/00, C12Q 1/68

(54) **METHOD OF ANALYZING MUTUAL INTERACTION BETWEEN PROTEIN AND MOLECULE**

(30) Priority: 31.08.1999 JP 24470499
(71) Applicant: MITSUBISHI CHEMICAL CORPORATION, Chiyoda-ku, Tokyo 100-0005 (JP)
(72) Inventor: YANAGAWA, Hiroshi, Aiko-gun, Kanagawa 243-0303 (JP); NEMOTO, Naoto, Machida-shi, Tokyo 194-0041 (JP)
(74) Representative: HOFFMANN - EITLE
(86) International application number: JP0005920
(87) International publication number: WO0116600

(57) **Abstract**

A method for analyzing an interaction of a protein and a molecule, which comprises (1) a step of bringing a C-terminus labeled protein into contact with a target molecule, and (2) a step of detecting a change of a signal which is generated on the basis of an interaction between the C-terminus labeled protein and the target molecule among signals generated by the C-terminus labeled protein or the target molecule; A method for identifying a molecule which interacts with a protein or identifying a protein which interacts with a molecule, which comprises: (1) a step of preparing a C-terminus labeled protein by labeling C-terminus of the protein, (2) a step of bringing the C-terminus labeled protein into contact with the target molecule, and (3) a step of judging that the C-terminus labeled protein and the target molecule have interaction when a change of a signal from the C-terminus labeled protein or the target molecule is detected wherein said change is caused by the contact of the C-terminus labeled protein and the target molecule.

SEQUENCE LISTING

<110> Mitsubishi Chemical Corporation

<120> A method of analyzing protein-molecule interaction

<130> A01369M

<150> JP P1999-244704

<151> 1999-08-31

<160> 16

<210> 1

<211> 88

<212> DNA

<213> Artificial Sequence

<220>

<223> Description of Artificial Sequence:synthetic

<400> 1

gatcccgcga aattaatacg actcactata gggagaccac aacggtttcc ctctagaaat 60

aattttgttt aactttaaga aggagatg 88

<210> 2

<211> 33

<212> DNA

<213> Artificial Sequence

<220>

<223> Description of Artificial Sequence:synthetic

<400> 2

gatcccgcga aattaatacg actcactata ggg 33

<210> 3

<211> 29

<212> DNA

<213> Artificial Sequence

<220>

<221> misc_feature

<222> (6)

<223> n is ribocytidylic acid.

<400> 3

ggaagncatg gtggcatctc cttcttaaa 29

<210> 4

<211> 29

<212> DNA

<213> Artificial Sequence

<220>

<221> misc_feature

<222> (6)

<223> n is ribocytidylic acid.

<400> 4

gcttcnaaac aaagcactat tgcactggc 29

<210> 5

<211> 30

<212> DNA

<213> Artificial Sequence

<220>

<223> Description of Artificial Sequence:synthetic

<400> 5

ccaatgctta atcagtgagg cacctatctc 30

<210> 6

<211> 32

<212> DNA

<213> Artificial Sequence

<220>

<223> Description of Artificial Sequence:synthetic

<400> 6

ggtctgacag ttaccaatgc ttaatcagtg ag 32

<210> 7

<211> 117

<212> DNA

<213> Artificial Sequence

<220>

<223> Description of Artificial Sequence:synthetic

<400> 7

gatcccgcga aattaatacg actcactata gggagaccac aacggtttcc ctctagaaat 60

aattttgttt aactttaaga aggagatgcc accatggttg agccccgcat ggagttc 117

<210> 8

<211> 31

<212> DNA

<213> Artificial Sequence

<220>

<223> Description of Artificial Sequence:synthetic

<400> 8

ggccccgcga aattaatacg actcactata g 31

<210> 9

<211> 40

<212> DNA

<213> Artificial Sequence

<220>

<223> Description of Artificial Sequence:synthetic

<400> 9

tgttgaattt gttatccatg gtggcatctc cttcttaaag                                40

<210> 10

<211> 25

<212> DNA

<213> Artificial Sequence

<220>

<223> Description of Artificial Sequence:synthetic

<400> 10

ctttaagaag gagatgccac catgg                                25

<210> 11

<211> 31

<212> DNA

<213> Artificial Sequence

<220>

<223> Description of Artificial Sequence:synthetic

<400> 11

gttgaattcg ttgtcagctt ttggtgcttg a   31

<210> 12

<211> 31

<212> DNA

<213> Artificial Sequence

<220>

<223> Description of Artificial Sequence:synthetic

<400> 12

gttgaattcg ttgtcagctt ttggtgcttg a                                    31

<210> 13

<211> 31

<212> DNA

<213> Artificial Sequence

<220>

<223> Description of Artificial Sequence:synthetic

<400> 13

gttgaattcg ttgtcagctt ttggtgcttg a                                    31

<210> 14

<211> 32

<212> DNA

<213> Artificial Sequence

<220>

<223> Description of Artificial Sequence:synthetic

<400> 14

tttgtagagc tcatccatgc catgtgtaat cc                                   32

<210> 15

<211> 39

<212> DNA

<213> Artificial Sequence

<220>

<223> Description of Artificial Sequence:synthetic

<400> 15

agatccgccg ccaccgttga atttgttgtc agcttttgg                            39

<210> 16

<211> 40

<212> DNA

<213> Artificial Sequence

<220>

<223> Description of Artificial Sequence:synthetic

<400> 16

ggtggcggcg gatctatgag taaaggagaa gaacttttca                    40

## Description

### Technical Field

The present invention relates to a method for identifying a molecule that interacts with a protein of which C-terminus is labeled. The present invention provides means for quickening analysis of functions of proteins encoded by nucleotide sequences of genes whose data have been rapidly accumulated recently, for example, analyses of protein-protein interaction, protein-nucleic acid interaction, protein-low molecular compound interaction and the like. The present invention also provides useful means for a method of rapidly analyzing a large number of data (realization of high-throughput), which is indispensable means for functional analysis of proteins.

### Background Art

With progress of molecular biology, it has become easy to analyze individual gene products, and as a result, it becomes possible to analyze life phenomena at a molecular level. For example, functions of proteins as gene products can be analyzed through biochemical functional studies based on analyses of protein-protein interactions, protein-nucleic acid interactions and the like.

As methods for analyzing protein-protein interactions, the yeast two hybrid method (Chien, C.T., et al., Proc. Natl. Acad. Sci. USA, 88, 9578-9582 (1991)), the phage display method (Smith, G.P., Science, 228, pp.1315-1317 (1985)), the GST-fusion protein pull down method, the immunocoprecipitation method and the like are known. Reactions are carried out in vitro for the GST-fusion protein pull down method and the immunocoprecipitation method, whereas interactions are detected in cells in the yeast two hybrid method.

As methods for analyzing protein-nucleic acid interactions, the electrophoresis mobility shift assay method (Revzin, A., et al., Anal. Biochem., 153, 172 (1986)), DNase I footprint method (Calas, D., et al., Nucleic Acids Res., 5, 3157 (1978)), the methylation buffering method and the like are known. In the electrophoresis mobility shift assay method, a fragment containing a target nucleic acid sequence is labeled with a radioisotope or the like to form a probe and brought into contact with a protein library, and then detected by polyacrylamide gel electrophoresis. When protein-nucleic acid interaction exists, a band with mobility different from that of the probe can be detected. A nucleic acid sequence that is recognized and bound by a protein, DNA binding domain in the protein and the like can be analyzed by said method.

A method of detecting an interaction by directly labeling a protein as a gene product is also known. This method is more excellent in sensitivity and accuracy compared with the aforementioned methods, because the analysis is based on the fact that signals from the labeled protein are changed by an interaction of the protein with other molecule.

As methods to directly label a protein, a radioactivity labeling method is generally used, in which an amino acid as a substrate which is labeled with a radioisotope such as ³⁵S, ³H and ¹⁴C is used for incorporation into a protein when a gene is expressed in a cell-free translation system or a living cell. However, the method requires special facilities and the like for safety control for the use of radioactive substance.

A method of using a labeled tRNA is known as a method that avoids a use of a radioactive compound. According to this method, a labeling substance such as biotin is covalently bonded to the ε-amino group of lysine as an amino acid and the product is bonded to tRNA having anticodon for lysine or the like through an ester bond to synthesize biotin-lysine-tRNA, and then the resulting product is introduced into a cell-free translation system to biotinylate a protein as a translation product. The protein produced by the method is transferred to a membrane after electrophoresis and allowed to generate chemiluminescence by using a fusion protein consisting of alkaline phosphatase and streptavidin. The translation product is identified based on the chemiluminescence by using an X-ray film or the like (Promega, Technical Bulletin, No.182, p.2 (1993)). However, this method has problems that the synthesized biotin-lysine-tRNA is extremely unstable (6 months at -70°C) and expensive. Furthermore, plural lysine side chains of the translated protein are modified with biotin, and moreover, the modified side chains are different in each molecule. As a result, plural kinds of translation products having functions and structures different from those of the original protein are obtained, although they are translated from the same gene, which results in extreme difficulty in functional analysis of the protein.

Furthermore, several methods for direct binding of a fluorescent substance to NH₂, SH or OH group of amino acid in a protein are known (PanVera, Fluorescence Polarization Applications Guide, Chapter 7 (1998)). However, in all of these methods, the chemical bonding is formed under a condition that denatures a protein, and it is highly likely that structure and function of the protein are significantly changed. Moreover, the fluorescent substance does not bind to a specific site of a side chain of the protein, but non-specifically binds to plural side chains. Therefore, plural kinds of proteins having structures and functions different from those of the original protein may exist, thereby accurate information cannot be obtained in the functional analysis of the protein. Furthermore, in this chemical modification method, purification of the target protein is essential.

The inventors of the present invention previously proposed methods of directly bonding a labeling substance to a C-terminus of a protein in a translation system using a nucleic acid derivative such as puromycin (Japanese Patent Application Nos. 10-133170 and 10-320093). These methods achieve labeling of a protein without damage of structure and function of the protein. However, these methods still have a problem of bonding efficiency of the labeling molecule at the C-terminus.

The aforementioned known methods for labeling a protein have a problem that labeled proteins cannot preserve its original structure and function, as well as problems of lack of safety, poor labeling efficiency and the like when they are used as methods to identify a molecule that interacts with a protein. Although the fluorescence depolarization method, the surface plasmon resonance method and the like have been available as means for rapidly analyzing intermolecular interactions, no satisfactory technique has been developed to effectively utilize said methods.

### Summary of the Invention

The inventors of the present invention conducted various studies to establish a method for identifying a molecule that interacts with a protein by using a C-terminus labeled protein. As a result, they found that a molecule that interacts with a protein was efficiently identifiable by using a C-terminus labeled protein, C-terminus labeled protein immobilized on a solid phase, which comprises a protein immobilized on a solid phase wherein a labeling substance is bonded to the C-terminus of the protein by means of a spacer, and a labeled molecule or the like. The present invention was achieved on the basis of these findings.

The following inventions are thus provided:
1. A method for analyzing an interaction between a peptide and a molecule, which comprises the following steps:
   (1) a step of bringing a C-terminus labeled protein into contact with a target molecule, and
   (2) a step of detecting a change of a signal which is caused on the basis of an interaction between the C-terminus labeled protein and the target molecule among signals generated by the C-terminus labeled protein or the target molecule.
2. The method according to the above 1, wherein the target molecule is a protein, a nucleic acid, a sugar chain, or a low molecular compound.
3. The method according to the above 1 or 2, wherein the target molecule is a labeled molecule which is labeled with a labeling substance.
4. The method according to the above 3, wherein the labeling substance is fluorescence labeling agent.
5. The method according to any one of the above 1 to 4, wherein the C-terminus labeled protein is prepared by expressing a nucleic acid comprising a coding region for the protein in a transcription and/or translation system to allow protein synthesis in the presence of a labeling agent which comprises a labeling moiety comprising a labeling substance and an acceptor moiety comprising a compound having an ability to bind to the C-terminus of a protein.
6. The method according to the above 5, wherein the acceptor moiety comprises a compound containing a chemical skeleton of puromycin, 3'-N-aminoacylpuromycin aminonucleoside, or 3'-N-aminoacyladenosine aminonucleoside, or an analogue of said compound.
7. The method according to the above 5 or 6, wherein the labeling agent comprises a compound in which the labeling moiety binds to the acceptor moiety via a spacer.
8. The method according to any one of the above 1 to 7, wherein either of the C-terminus labeled protein or the target molecule is bound to a solid phase, or said method wherein a mode of the binding to the solid phase is either of the following a or b:
   a: each substance of either of the C-terminus labeled protein or the target molecule is immobilized on a base plate with an address thereof, or
   b: each substance of either of the C-terminus labeled protein or the target molecule is immobilized on a bottom surface of each well of a microplate having plural wells.
9. The method according to the above 8, wherein the C-terminus labeled protein is bound to the solid phase via the labeled moiety or a moiety other than the labeled moiety which constitutes said protein.
10. The method according to the above 9, wherein the labeled moiety constituting the C-terminus labeled protein comprises a molecule having an ability to specifically bind to a particular polypeptide, wherein said molecule is bound to the solid phase via a linkage with said particular polypeptide bound to a surface of the solid phase.
11. The method according to the above 10, wherein a combination of the particular polypeptide and the molecule having an ability to specifically bind to the particular polypeptide is selected from the group consisting of combinations of biotin binding protein/biotin, maltose binding protein/maltose, G protein/guanine nucleotide, polyhistidine peptide/metal ion, glutathione-S-transferase/glutathione, DNA binding protein/DNA, antibody/antigen molecule, calmodulin/calmodulin binding peptide, adenosine triphosphate (ATP) binding protein/ATP and estradiol receptor protein/estradiol.
12. The method according to any one of the above 1 to 11, wherein the change of the signal is measured by one or more methods selected from the group consisting of surface plasmon resonance method, evanescent field imaging method, fluorescence imaging analysis method, enzyme linked immunosorbent assay, fluorescence depolarization method, and fluorescence correlation spectroscopy.
13. The method according to any one of the above 1, 2 and 5 to 11, wherein the C-terminus labeled protein is bound to the solid phase via the labeled moiety constituting the protein, wherein the target molecule is an unlabeled molecule or a molecule labeled with a labeling substance, and wherein the change of the signal is measured by one or more of methods selected from the group consisting of surface plasmon resonance method, evanescent field imaging method, fluorescence imaging analysis method, and enzyme linked immunosorbent assay.
14. The method according to the above 13, wherein the labeled molecule is the target molecule labeled with a fluorescence labeling agent.
15. The method according to any one of the above 1, 2 and 5 to 11, wherein the C-terminus labeled protein is bound to the solid phase via a moiety other than the labeled moiety which constitutes the protein, wherein the target molecule is an unlabeled molecule or a molecule labeled with a labeling substance, and wherein the change of the signal is measured by one or more of methods selected from the group consisting of surface plasmon resonance method, evanescent field imaging method, fluorescence imaging analysis method and enzyme linked immunosorbent assay.
16. The method according to the above 15, wherein the labeled molecule is the target molecule labeled with a fluorescence labeling agent.
17. The method according to any one of the above 1, 2 and 5 to 11, wherein the target molecule is bound to the solid phase, and wherein the change of the signal generated from the labeled moiety of the C-terminus labeled protein is measured by one or more of methods selected from the group consisting of surface plasmon resonance method, evanescent field imaging method, fluorescence imaging analysis method, and enzyme linked immunosorbent assay.
18. The method according to any one of the above 1, 2 and 5 to 11, wherein the C-terminus labeled protein and the target molecule are not bound to the solid phase but exist in a phase of a solution, wherein the target molecule is an unlabeled molecule or a molecule labeled with a labeling substance, and wherein the change of the signal is measured by fluorescence depolarization method and/or fluorescence correlation spectroscopy.
19. A labeling agent for a protein comprising a labeling moiety and an acceptor moiety, wherein the labeling moiety is a molecule having an ability to specifically bind to a particular polypeptide, and the acceptor moiety is a compound having an ability to bind to a C-terminus of a protein.
20. The agent according to the above 19, wherein the labeling moiety is bound to the acceptor moiety via a spacer.
21. The agent according to the above 19 or 20, wherein the spacer is either polyethylene or polyethylene glycol.
22. The agent according to any one of the above 19 to 21, wherein the labeling moiety comprises one or more molecules selected from the group consisting of biotin, maltose, guanine nucleotide, metal ion, glutathione, protein binding DNA, antigen molecule, calmodulin binding peptide, ATP, and estradiol.
23. The agent according to any one of the above 19 to 22, wherein the acceptor moiety comprises a compound containing a chemical skeleton of puromycin, 3'-N-aminoacylpuromycin aminonucleoside or 3'-N-aminoacyladenosine aminonucleoside, or an analogue of said compound.
24. An immobilized protein characterized in that a C-terminus labeled protein is bound to a solid phase via a labeled moiety which constitutes the protein.
25. The immobilized protein according to the above 24, wherein the C-terminus labeled protein is prepared by expressing a nucleic acid containing a coding region for the protein in a transcription and/or translation system to allow protein synthesis in the presence of a labeling agent comprising a labeling moiety containing a labeling substance and an acceptor moiety containing a compound having an ability to bind to a C-terminus of a protein.
26. The immobilized protein according to the above 24 or 25, wherein the labeling moiety of the labeling agent is covalently bound to the acceptor moiety via a spacer.
27. The immobilized protein according to any one of the above 24 to 26, wherein the labeling moiety is a molecule having an ability to specifically bind to a particular polypeptide and wherein the molecule binds to the particular polypeptide which is bound to the solid phase.
28. The immobilized protein according to any one of the above 24-27, wherein the acceptor moiety comprises a compound having a chemical skeleton of puromycin, 3'-N-aminoacylpuromycin aminonucleoside or 3'-N-aminoacyladenosine aminonucleoside, or an analogue of said compound.
29. The immobilized protein according to any one of the above 24 to 28, wherein a combination of the particular molecule constituting the labeling moiety and the molecule having an ability to specifically bind to the molecule is a combination selected from the group consisting of combinations of biotin binding protein/biotin, maltose binding protein/maltose, G protein/guanine nucleotide, polyhistidine peptide/metal ion, glutathione-S-transferase/glutathione, DNA binding protein/DNA, antibody/antigen molecule, calmodulin/calmodulin binding peptide, ATP binding protein/ATP, and estradiol receptor protein/estradiol.
30. A protein chip comprising an assembly of immobilized proteins according to any one of the above 24-29.
31. An apparatus for producing the protein chip according to the above 30, which comprises means for holding a base plate whose surface is bound with an adapter protein, means for introducing a C-terminus labeled protein into the base plate, and means for washing the base plate.
32. An apparatus for simultaneous analyses of multiple test samples by applying a method according to any one of the above 10, 11, 13 and 14, which comprises means for holding a chip, means for adhering a target molecule to the chip, means for washing the chip, and means for measuring a signal from the C-terminus labeled protein and detecting a change of the signal based on an interaction between the C-terminus labeled protein and the target molecule.
33. A method for identifying a molecule which interacts with a protein or identifying a protein which interacts with a molecule, which comprises:
   a. the following steps:
      (1) a step of preparing a C-terminus labeled protein by labeling C-terminus of the protein,
      (2) a step of bringing the C-terminus labeled protein into contact with the target molecule, and
      (3) a step of judging that the C-terminus labeled protein and the target molecule have interaction when a change of a signal from the C-terminus labeled protein or the target molecule is detected wherein said change is caused by the contact of the C-terminus labeled protein and the target molecule; and
   b. following the above steps (1) to (3),
      (4) a step of identifying the protein and the target molecule which are judged to have interaction.
34. A method for identifying a molecule which interacts with a particular protein or identifying a protein which interacts with a particular molecule, which comprises:
   a. the following steps:
      (1) a step of bringing a C-terminus labeled protein comprising the protein into contact with the target molecule, and
      (2) a step of judging that the C-terminus labeled protein and the target molecule have interaction when a change of a signal from the C-terminus labeled protein or the target molecule is detected wherein said change is caused by the contact of the C-terminus labeled protein and the target molecule; and
   b. following the above steps (1) and (2),
      (3) a step of identifying the protein and the target molecule which are judged to have interaction.
35. A method for screening a molecule which interacts with a protein or screening a protein which interacts with a molecule, which comprises the following steps:
   (1) a step of bringing a C-terminus labeled protein comprising the protein into contact with the target molecule, and
   (2) a step of judging that the C-terminus labeled protein and the target molecule have interaction when a change of a signal from the C-terminus labeled protein or the target molecule is detected wherein said change is caused by the contact of the C-terminus labeled protein and the target molecule.
36. The molecule screened by the method according to the above 35 which interacts with said protein or the protein screened by the method according to the above 35 which interacts with said molecule.
37. A C-terminus labeled protein for use in the method for identifying a molecule which interacts with a protein according to the above 33 or 34.
38. A C-terminus labeled protein for use in the screening method according to the above 35.

### Brief Explanation of the Drawings

Fig. 1 shows chemical structures of puromycin and derivatives thereof, including I: Puromycin, II: rCpPur, III: dCpPur, and IV: dUpPur.

Fig. 2 shows chemical structures of puromycin bound to a fluorescent substance, including I: Fluorpur and II: Fluorthiopur.

Fig. 3 is a schematic view showing a C-terminus protein immobilized on a solid phase in which a ligand as a labeling moiety is bound to an adapter protein immobilized to the solid phase.

Fig. 4 shows results of measurement of interaction between the domain B and human IgG by using a fluorescence depolarization measurement apparatus, which are represented as changes of polarization degree for each human IgG concentration.

Fig. 5 is a photograph of fluorescence intensity of a protein labeled with biotin/puromycin (Biotin-puro) which is depicted on a display of a fluorescence imaging analyzer.

Fig. 6 is a photograph showing intensity of interaction between B domain immobilized on a solid phase and human IgG represented as fluorescence intensity which is depicted on a display of a fluorescence imaging analyzer.

### Best Mode for Carrying out the Invention

The present invention will be explained in more detail.

### (1) C-Terminus labeled protein

### (1-1) Constitution of C-terminus labeled protein

One of characteristic features of the method for analyzing an interaction between a protein and a molecule according to the present invention is to use a C-terminus labeled protein. The C-terminus labeled protein is constituted by a protein moiety and a labeled moiety in which C-terminus of the protein is labeled with a labeling agent.

The "protein moiety" means a moiety of a protein used as an object of analysis for an interaction, of which function is known or unknown, and presence or absence of interaction between the aforementioned protein moiety and the target molecule described later is determined.

This protein moiety may be any of natural proteins or mutants thereof and artificial proteins or mutants thereof. The natural proteins include a library of variety of proteins, which are transcribed and translated from a cDNA library derived from organs, tissues or cells of various organisms. The artificial proteins include sequences comprising a combination of a whole or partial sequence of any natural protein and a random amino acid sequence.

### (1-2) Labeling agent

The "labeling agent" comprises a "labeling moiety" containing a labeling substance and an "acceptor moiety" containing a compound having an ability to bind to C-terminus of a protein. The labeling moiety and the acceptor moiety are bonded by means of a chemical bond. The labeling moiety and the acceptor moiety may be directly bonded with a chemical bond, or they may be chemically bonded via a spacer.

The labeling substance is usually selected from non-radioactive labeling substances such as fluorescent substances. The fluorescent substances have a free functional group (e.g., carboxyl group that can be converted into an active ester, hydroxyl group or amino group that can be converted into phosphoamidite and the like) and may be any fluorescent dyes that can be linked to the aforementioned nucleic acid derivatives such as puromycin or a puromycin-like compound, whose examples include various fluorescent dyes such as fluorescein-type dyes, rhodamine-type dyes, eosine-type dyes and NBD-type dyes. Type and size of the compound as the labeling moiety is not particularly limited so long as the compound is a non-radioactive labeling substance or a compound which can be labeled, such as a molecule having an ability to bind to a particular polypeptide which will be described later (referred occasionally as "ligand"), a protein, a peptide, a saccharide, a lipid, a dye, and a polyethylene glycol.

As the labeling substance, those suitable for the measurement of a change of a signal generated by interaction between the C-terminus labeled protein and the target molecule may be appropriately used.

As the "acceptor moiety" that constitutes the labeling agent, a nucleic acid derivative is usually used. Although the nucleic acid derivative is not limited so long as the derivative has an ability to bind to a C-terminus of a protein synthesized by protein synthesis (translation) performed in a cell free protein synthesis system or a living cell, the derivative may be chosen from derivatives whose 3' end have chemically similar skeleton to an aminoacyl-tRNA. Typical compounds include amide bond-containing compounds such as puromycin, 3'-N-aminoacylpuromycin aminonucleoside (PANS-amino acid) such as PANS-Gly whose amino acid moiety is glycine, PANS-Val whose amino acid moiety is valine, PANS-Ala whose amino acid moiety is alanine, and other PANS-amino acid compounds each of which amino acid moiety is every kind of amino acids,.

3'-Aminoacyladenosine aminonucleoside (AANS-amino acid) may also be used, in which the amino group of 3-aminoadenosine and carboxyl group of an amino acid are linked with an amide bond formed by dehydration condensation, such as AANS-Gly whose amino acid moiety is glycine, AANS-Val whose amino acid moiety is valine, AANS-Ala whose amino acid moiety is alanine and other AANS-amino acid compounds each of which amino acid moiety is every kind of amino acids.

Nucleosides and compounds in which a nucleoside and an amino acid are bonded via an ester bond may also be used. Furthermore, any compounds are encompassed within the nucleic acid derivatives used in the methods of the present invention in which a nucleic acid or a substance having a chemical skeleton analogous to that of a nucleic acid and a base is chemically bound to a substance having a chemical skeleton analogous to that of an amino acid.

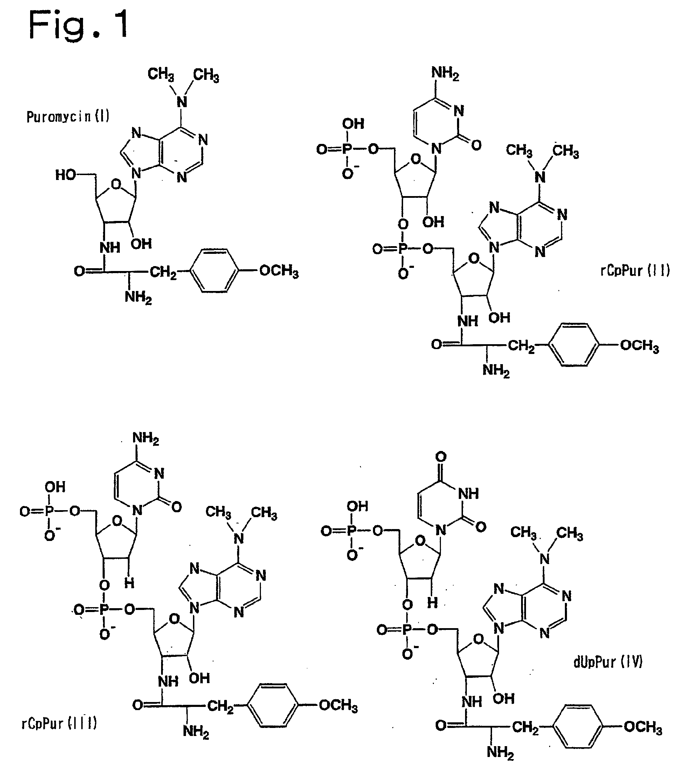

More preferred acceptor moieties include compounds in which puromycin, PANS-amino acid, or AANS-amino acid is bound to a nucleoside via a phosphate group. Among such compounds, particularly preferred are puromycin derivatives such as puromycin (Fig. 1, I), ribocytidylpuromycin (rCpPur: Fig. 1, II, deoxycytidypuromycin (dCpPur: Fig. 1, III) and deoxyuridylpuromycin (dUpPur: Fig. 1, IV).

The labeling agent can be produced by binding the aforementioned labeling moiety and acceptor moiety, optionally via a spacer, by a method for chemical bond formation which is known, per se. Specifically, the agent can be produced by, for example, binding the aforementioned acceptor moiety protected with a suitable protective group to a solid phase carrier, successively binding a spacer phosphoamidite as the spacer and phosphoamidite bound with a fluorescent substance or the like as a labeling moiety by using a nucleic acid synthesizer, and then eliminating the protective group. According to the kinds of the aforementioned moieties or type of binding, the chemical synthesis can be carried out by a liquid phase synthesis or a combination of a liquid phase synthesis and a solid phase synthesis. Further, in order to bind a metal ion such as nickel ion as the labeling moiety, the binding can be achieved by using a regent having a chelating property to which a metal ion can coordinate, such as nitrilotriacetatic acid and iminodiacetic acid.

As the spacer that connects the labeling moiety and the acceptor moiety, a polymer substance such as polyethylene and polyethylene glycol may be used, and polyethylene glycol may preferably be used.

### (1-3) Preparation of C-terminus labeled protein

The method of preparing the C-terminus labeled protein used in the present invention is not particularly limited. For example, a coding region which encodes the aforementioned protein moiety is ligated to a site under control of a promoter region derived from a virus such as T7 or a cell in the presence of the aforementioned labeling agent and transcribed to synthesize mRNA. As the coding region DNA, a sequence of which stop codon is deleted is preferably used to higher labeling efficiency by several tens of times. mRNA obtained from a living body by a method known, per se, can also be used. These mRNAs can be prepared in a translation system by expression for synthesis of a protein.

Examples of the translation system to be used include a cell free translation systems or a living cells. The cell free translation systems or the living cells are not limited so long as they perform protein synthesis by addition or introduction of a nucleic acid coding for a protein. As the cell free translation system, cell free translation systems constituted by an extract of prokaryote or eukaryote, for example, *Escherichia coli,* rabbit reticulocyte, wheat germ extract and the like can be used. As the living cell translation system, cells of prokaryotes or eukaryotes, for example, a cell of *Escherichia coli* and the like may be use. When a cell free translation system is used and the nucleic acid applied is DNA, RNA synthesized by transcription according to a method known per se by using an RNA polymerase or the like is introduced as a template.

A labeling substance can be bound to the C-terminus of the synthesized protein via the acceptor moiety by allowing a labeling agent to exist in the translation system at a suitable concentration. The concentration of the labeling agent to be exist may vary depending on the labeling agent, nucleic acid or translation system which are practically used. Generally, a range of a final concentration of 200 to 0.1 µM may be preferably applied.

A suitable concentration of the labeling agent can be chosen by introducing the labeling agent into translation system at various concentrations together with a template nucleic acid to be used, separating a synthesized protein by a suitable method, and then measuring intensity of signals generated by the protein and choosing a concentration of the labeling agent which is introduced in a system that gives the highest value.

An specific example of the concentration of the labeling agent chosen as described above includes 0.1 to 1 µ M when the nucleic acid used as a template comprises a whole length DNA comprising the T7 promoter and the coding region for thioredoxin, the labeling agent is a conjugate of puromycin and fluorescein, and the translation and extract of rabbit reticulocytes is used as the translation system. When wheat germ extract is used as the translation system, an example includes 10 to 50 µM.

The concentration of the nucleic acid derivative bound with the labeling substance may vary depending on the RNA, labeling agent, nucleic acid derivative, translation system and the like. Those skilled in the art can suitably determine the concentration as follows.

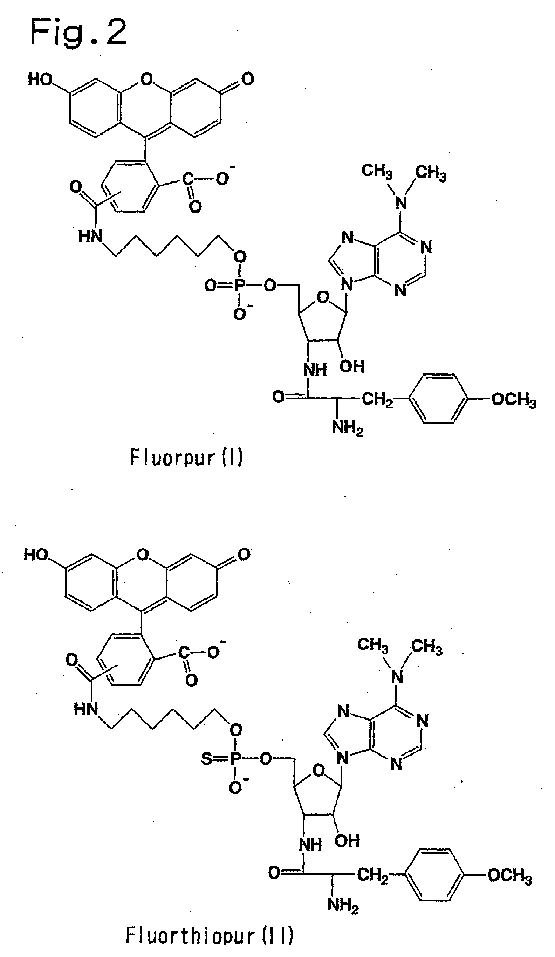

A nucleic acid derivative bound with a labeling substance, for example, fluorescenylpuromycin, (Fluorpur: Fig. 2, I) is added to the aforementioned system for preparing C-terminus labeled protein at different concentrations, and each resulting translation product is separated by using SDS polyacrylamide electrophoresis or the like. Then, intensities of the signals generated by the labeling substances bonded to the C-termini are measured, and a concentration giving the highest value of the signal intensity is chosen. Specifically, when a protein is synthesized by using the whole length DNA of the coding region for thioredoxin under the control of the T7 promoter in a cell free translation system of *Escherichia coli* in the presence of Fluorpur or Fluorthiopur (Fig. 2, II), an optimal concentration of Fluorpur or Fluorthiopur is 0.1 to 1 µM. An optimal concentration is 0.3 to 50 µM when extract of rabbit reticulocytes is used, and 10-50 µM when wheat germ extract is used.

When a live cell is used as the translation system, the C-terminus labeled protein synthesized as described above can be isolated by lysing the cell by a method known per se and removing unreacted labeling agent by gel filtration (using, for example, Bio-Spin 6, BIO-Rad) or the like. When a cell free translation system is used, a product is isolated by removing the unreacted labeling agent by gel filtration.

Further, in the present invention, the C-terminus labeled protein may be bound to a solid phase. Examples of the method of binding to a solid phase include a method of binding via the labeling moiety and a method of binding via a moiety other than the labeling moiety.

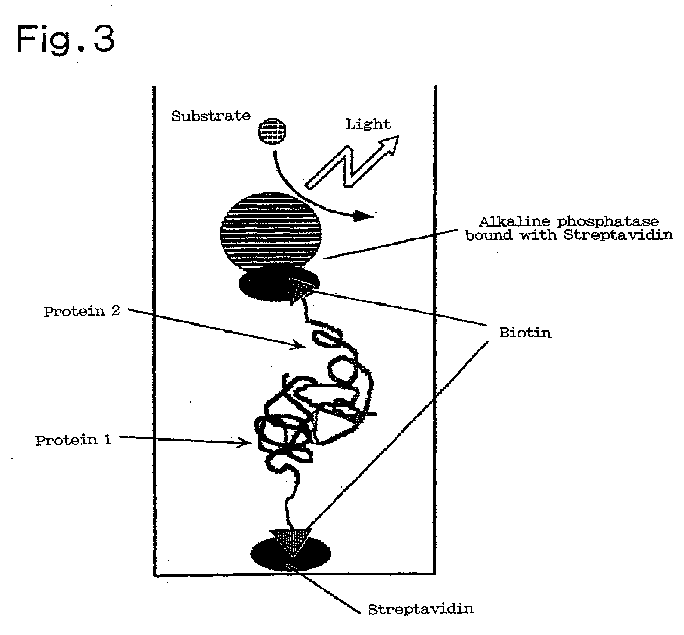

The labeling moiety used for the binding via the labeling moiety is a molecule that specifically binds to a particular polypeptide (hereinafter also referred to as a "ligand"), and the particular polypeptide that binds to the ligand (hereinafter also referred to as "adapter protein") is bound to a surface of the solid phase (Fig. 3). The adapter protein also includes binding proteins, receptor proteins constituting receptors and antibodies.

Examples of the combination of adapter protein/ligand include, for example, biotin binding protein such as avidin and streptavidin/biotin, maltose binding protein/maltose, G protein/guanine nucleotide, polyhistidine peptide/metal ion such as nickel ion or cobalt ion, glutathione-S-transferase/glutathione, DNA binding protein/DNA, antibody/antigen molecule (epitope), calmodulin/calmodulin binding peptide, ATP binding protein/ATP, various receptor proteins/ligands thereof such as estradiol receptor protein/estradiol and the like.

Among them, biotin binding protein such as avidin and streptavidin, maltose binding protein/maltose, polyhistidine peptide/metal ion such as nickel ion or cobalt ion, glutathione-S-transferase/glutathione, antibody/antigen molecule (epitope) and the like are preferred as the combination of adapter protein/ligand, and the combination of streptavidin/biotin is most preferred. These binding proteins per se are known, and DNA coding for these proteins have been already cloned.

For binding of the adapter protein to a surface of solid phase, a method known per se can be used. Specifically, for example, methods utilizing tannic acid, formalin, glutaraldehyde, pyruvic aldehyde, bis-diazotized benzizone, toluene-2,4-diisocyanate, amino group, carboxyl group, or hydroxyl group or amino group may be used.

When the protein is bound to the solid phase via a moiety other than the labeling moiety, the binding can be usually formed by a known method used for binding a protein to a solid phase, for example, methods utilizing tannic acid, formalin, glutaraldehyde, pyruvic aldehyde, bis-diazotized benzizone, toluene-2,4-diisocyanate, amino group, carboxyl group, or hydroxyl group or amino group.

### (2) Target molecule

In the present invention, "target molecule" means a molecule that interacts with the C-terminus labeled protein. Specific examples include a protein, nucleic acid, sugar chain, low molecular compound or the like.

The proteins are not particularly limited so long as they have an ability to interact with the C-terminus labeled protein, and the proteins may be whole length proteins or a partial peptide containing an active site for binding. Proteins may be those with known or unknown amino acid sequence and functions. Synthesized peptide chains, proteins purified from a living body, proteins translated from a cDNA library by using a suitable translation system or the like and then purified and the like may also be used as the target molecule. The synthesized peptide chain may a glycoprotein in which a sugar chain is bound to the peptide chain. Among them, a purified protein with known amino acid sequence and a protein translated from a cDNA library or the like and purified by a suitable method can be preferably used.

The nucleic acids are not particularly limited so long as they have an ability to interact with the C-terminus labeled protein, and either DNA or RNA may be used. Nucleic acids may be those with known or unknown nucleotide sequence or function. Preferably, nucleic acids with known function and sequence which have an ability to bind to a protein, or a nucleic acid digested and isolated from a genomic library or the like by using a restriction enzyme or the like may be used.

The sugar chains are not particularly limited so long as they have an ability to interact with the C-terminus labeled protein, and may be those with known or unknown sugar sequence or function. Preferably, sugar chains which are already isolated and analyzed and sugar sequence and function thereof are known may preferably be used.

The low molecular compounds are not particularly limited so long as they have an ability to interact with the C-terminus labeled protein. Compounds with unknown function or those with known ability to bind to a protein may also be used.

The "interaction" of the target molecules with the C-terminus labeled protein generally means an action based on an intramolecular force generated by at least one of covalent bond, hydrophobic bond, hydrogen bond, van der Waals bond and bond by electrostatic force between the protein and the target molecule. However, the term should be construed in its widest sense, and the term should not be limitatively construed in any sense. The covalent bond includes a coordinate bond and dipole bond. Further, the bond by electrostatic force includes electric repulsion in addition to electrostatic bond. Bonding reactions, synthetic reactions and decomposition reactions caused as a result of the aforementioned action also fall within the scope of the interaction.

Specific examples of the interaction include association and dissociation between an antigen and an antibody, association and dissociation between a protein receptor and a ligand, association and dissociation between an adhesion molecule and a partner molecule, association and dissociation between an enzyme and a substrate, association and dissociation between a nucleic acid and a protein binding thereto, association and dissociation between proteins in a transmission system, association and dissociation between a glycoprotein and a protein, and association and dissociation between a sugar chain and a protein.

The target molecule used may be labeled with a labeling substance depending on an embodiment and then used. The labeling substance is usually selected from non-radioactive labeling substances such as fluorescent substances. The fluorescent substances may be a variety of fluorescent dyes which have a free functional group (e.g., carboxyl group, hydroxyl group, amino group and the like) and can be linked to the aforementioned target substance, for example, any of fluorescein-type dyes, rhodamine-type dyes, eosine-type dyes, NBD-type dyes and the like. Kind and size of the compound are not particularly limited so long as the compound enables labeling such as dyes.

As the labeling substance, those suitable for the measurement of a change of a signal based on the interaction between the target molecule and the C-terminus labeled protein may be appropriately used.

The aforementioned labeling substance can be bound to the target molecule by using a suitable method known per se. Specifically, when the target molecule is a protein, for example, the method for labeling the C-terminus described in the above section 1. Further, when the target molecule is a nucleic acid, labeling may be easily achieved by a method in which PCR is performed by using oligo DNA primers to which the labeling substance is bound beforehand via a covalent bond or the like.

Further, the target molecule used in the present invention may be bound to a solid phase depending on embodiments. Examples of the method of binding to a solid phase include a method of binding via the labeling substance and a method of binding via a moiety other than the labeling substance.

When the binding is formed via the labeling substance, the labeling substance used is a ligand, and an adapter protein that binds to the ligand is bound to the surface of a solid phase.

Examples of the combination of adapter protein/ligand include, for example, biotin binding protein such as avidin and streptavidin/biotin, maltose binding protein/maltose, G protein/guanine nucleotide, polyhistidine peptide/metal ion such as nickel ion or cobalt ion, glutathione-S-transferase/glutathione, DNA binding protein/DNA, antibody/antigen molecule (epitope), calmodulin/calmodulin binding peptide, ATP binding protein/ATP, various receptor proteins/ligands thereof including estradiol receptor protein/estradiol and so forth.

Among them, preferred examples of the combination of adapter protein/ligand include biotin binding protein such as avidin and streptavidin, maltose binding protein/maltose, polyhistidine peptide/metal ion such as nickel ion or cobalt ion, glutathione-S-transferase/glutathione, antibody/antigen molecule (epitope) and the like, and the combination of streptavidin/biotin is most preferred. These binding proteins per se are known, and DNA coding for these proteins have been already cloned.

For binding of the adapter protein to a surface of solid phase, a method that is known per se can be applied. Specifically, applicable methods include, for example, methods utilizing tannic acid, formalin, glutaraldehyde, pyruvic aldehyde, bis-diazobenzizone, toluene-2,4-diisocyanate, amino group, carboxyl group that can be converted into an active ester, hydroxyl group or amino group that can be converted into phosphoamidite or the like.

When the protein is bound to the solid phase via a moiety other than the labeling substance, the binding can be usually attained by a known method used for binding a protein, nucleic acid, sugar chain, low molecular weight compound or the like on a solid phase. Specifically, applicable methods include, for example, methods utilizing tannic acid, formalin, glutaraldehyde, pyruvic aldehyde, bis-diazobenzizone, toluene-2,4-diisocyanate, amino group, carboxyl group that can be converted into an active ester, hydroxyl group or amino group that can be converted into phosphoamidite or the like.

### (3) Method for measurement of a change of a signal

The C-terminus labeled protein and the target molecule obtained as described above, suitably combined depending on the type of the labeling substance or the scheme of the immobilization on the solid phase, are brought into contact with each other, and an interaction between the C-terminus labeled protein and the target molecule is analyzed by measuring and detecting a change of a signal based on the interaction between said two molecules among the signals generated from the C-terminus labeled protein or the target molecule.

The "measurement" is a means to collect a change of a signal used for the analysis, and the term should not be limitatively construed in any sense. Examples of applicable measuring methods include, for example, the surface plasmon resonance method, evanescent field molecule imaging method, fluorescence imaging analysis method, enzyme linked immunosorbent assay, fluorescence depolarization method, fluorescence correlation spectroscopy and the like.

### (3-1) Surface plasmon resonance method

The surface plasmon resonance is a method in which surface plasmon is excited by a molecule that interacts at a metal/liquid interface and the resulting plasmon is measured based on change in intensity of reflected light (Cullen, D.C., et al., Biosensors, 3 (4), 211-225 (1987-88)). When the measurement or analysis of a protein-molecule interaction is performed by using this method, the C-terminus labeled protein is required be immobilized on a solid phase by the aforementioned method. However, labeling of the target molecule is not required.

As a base plate for immobilizing the C-terminus labeled protein on a solid phase, a transparent base plate composed of glass or the like, on which a thin film of a metal such as gold, silver and platinum is formed, may be used. The transparent base plates may be any type of base plates so long as they are usually used for surface plasmon resonance apparatuses. The base plate may generally be composed of glass as a material transparent to a laser light. A glass base plate having a thickness of about 0.1 to 5 mm may be used. The thickness of the thin metal film is suitably about 100 to 2000Å. As such base plates for surface plasmon resonance apparatuses, commercially available products can also be used. The C-terminus labeled protein can be immobilized as a solid phase on the aforementioned base plate by the aforementioned method.

In the aforementioned method, any methods may be applied to bring the target compound into contact with the C-terminus labeled protein so long as the method can achieve contact of the two molecules at a sufficient degree for their interaction. Preferably, a method is applied in which the C-terminus labeled protein immobilized on a solid phase is brought into contact with a solution containing dissolved target molecule in a buffer ordinarily used for biochemical purposes at an appropriate concentration.

These processes may be performed by using a commercially available surface plasmon resonance apparatus, such as BIAcore 2000 (Pharmacia Biosensor).

After the both molecules are brought into contact with each other, the interaction between the C-terminus labeled protein immobilized on a solid phase and the target molecule can be analyzed by measuring changes with time of relative intensity of reflected light of each molecule by using a surface plasmon resonance apparatus that is known per se.

As a method for simultaneously performing multiple analyses by the above method, for example, applicable methods include a method in which each kind of plural C-terminus labeled proteins is immobilized on the aforementioned base plate used for a surface plasmon resonance apparatus with an address thereof, a method in which plural kinds of target molecules are brought into contact with a single kind of the C-terminus labeled protein immobilized on a solid phase or the like.

### (3-2) Evanescent field molecule imaging method

The evanescent field molecule imaging method is disclosed in Funatsu, T., et al., Nature, 374,555-559, (1995) and the like. The method comprises the step of bringing a molecule immobilized on a solid phase of transparent body such as glass is brought into contact with a second molecule as a solution, then irradiating the transparent body with a light source such as a laser light at such an angle that an evanescent field is generated, and then measuring or analyzing the generated evanescent light by a detector. These procedures can be performed by using an evanescent field fluorescence microscope that is known per se.

When an interaction between a protein and a molecule is measured or analyzed by using this method, either of the C-terminus labeled protein or the target molecule is required to be immobilized on a solid phase by the method described above. When the target molecule is immobilized on a solid phase, its labeling is not required, whereas the target molecule needs to be labeled with the aforementioned labeling substance when used without immobilization on a solid phase.

As a base plate for immobilizing the C-terminus labeled protein or the target molecule on a solid phase, a base plate made of a material such as glass or the like is used, and quartz glass is preferably used. Further, for the purpose of preventing scatter of laser light and the like, a base palate whose surface is subjected to ultrasonication washing is preferred.

As methods of bringing the C-terminus labeled protein or labeled target molecule not immobilized on a solid phase into contact with a molecule immobilized on a solid phase in the aforementioned methods, any methods may be applied so long as they can achieve contact of the two molecules in a sufficient degree for their interaction. Preferably, a method may be applied in which a solution is prepared by dissolving the C-terminus labeled protein or the labeled target molecule not immobilized on a solid phase in a buffer ordinarily used for biochemical purposes at an appropriate concentration and then the solution is dropped onto a surface of the solid phase.

After the two molecules are brought into contact with each other, a molecule that interacts with the molecule immobilized on the solid phase can be identified by measuring fluorescence excited by the evanescent field illumination using a detector such as a CCD camera.

As a method for simultaneously performing multiple analyses by the aforementioned method, for example, a method may be used in which each kind of plural C-terminus labeled proteins or plural labeled target molecule is immobilized on the aforementioned base plate with its address.

### (3-3) Fluorescence imaging analysis method

The fluorescence imaging analysis is a method comprising the step of bringing a labeled molecule into contact with a molecule immobilized on a solid phase and measuring or analyzing fluorescence by using a commercially available fluorescence imaging analyzer which is emitted from the labeled molecule that remains on the molecule immobilized on the solid phase due to an interaction of the both molecules.

When measurement or analysis of an interaction between a protein and a molecule is performed by using the aforementioned method, either of the C-terminus labeled protein or the target molecule is required to be immobilized on a solid phase by the method mentioned above. When the target molecule is immobilized on a solid phase, either of the labeled or non-labeled target molecule may be used. When the target molecule is used without immobilization on a solid phase, the molecule is required to be labeled with the aforementioned labeling substance. As the C-terminus labeled proteins, either of those immobilized via the labeled moiety or those immobilized via a moiety other than the labeled moiety may be used.

As a base plate for immobilizing the C-terminus labeled protein or the target molecule on a solid phase, a nitrocellulose membrane, nylon membrane, plastic microplate and so forth, which are usually used for immobilizing a protein, nucleic acid and the lime may be used.

As methods for bringing the labeled target molecule or the C-terminus labeled protein into contact with a molecule immobilized on a solid phase in the aforementioned method, any methods may be employed so long as they can achieve contact of the two molecules in a sufficient degree for their interaction. Preferably, a method is used in which a solution is prepared by dissolving the labeled target molecule or the C-terminus labeled protein in a buffer ordinarily used for biochemical purpose at an appropriate concentration and then the solution is brought into contact with a surface of a solid phase.

After the two molecules are brought into contact with each other, and preferably after a process is applied to wash excess labeled target molecule or C-terminus labeled protein with the same buffer or the like, and then by using a marketed imaging analyzer, a molecule that interacts with the molecule immobilized on a solid phase can be identified by measuring or analyzing a fluorescence signal generated from the labeling substance of the target molecule or C-terminus labeled protein remaining on the solid phase, or a mixed signal comprising fluorescence generated by the labeled molecule immobilized on the solid phase and fluorescence generated by the labeled molecule remaining on the solid phase.

As methods for simultaneously performing multiple analyses by the aforementioned method, for example, a method in which each kind of plural C-terminus labeled proteins or labeled or unlabeled target molecules is immobilized on the aforementioned solid phase with its address, or a method in which plural kinds of C-terminus labeled proteins or labeled target molecules not immobilizedon a solid phase are brought into contact with a single kind of C-terminus labeled protein or labeled or unlabeled target molecule may be used. When plural kinds of C-terminus labeled proteins or labeled target molecules are brought into contact, the molecule remained on the solid phase can be identified by dissociating and obtaining said molecule on the basis of difference of buffer concentration or the like, and then analyzing the molecule by a known method.

### (3-4) Enzyme linked immunosorbent assay

The enzyme linked immunosorbent assay (ELISA, Crowther J.R., Methods in Molecular Biology, 42 (1995)) is a method in which a solution containing an antibody is brought into contact with an antigen immobilized on a solid phase, and by using a commercially available detector (ELISA reader), fluorescence generated by a labeled molecule that specifically reacts with the antibody remaining on the antigen immobilized on the solid phase (IgG and the like) based on interaction of the two molecules (antigen/antibody reaction) or a signal generated from a dye as a substrate of an enzyme which bind to a labeled molecule.

When measurement or analysis of an interaction between a protein and a molecule is performed by applying the aforementioned method, the C-terminus labeled protein that serves as an antigen is required to be immobilized on a solid phase by the method described above. The target molecule as an antibody is required to be labeled with the aforementioned labeling substance.

As a base plate for immobilizing the C-terminus labeled protein as an antigen on a solid phase, a microplate made of plastics ordinarily used for ELISA and the like can also be used.

As methods for bringing the labeled target molecule as an antibody into contact with the molecule immobilized on the solid phase, any methods may be employed so long as they can achieve contact of the two molecules in a sufficient degree for their interaction. Preferably, a method is used in which a solution is prepared by dissolving the labeled target molecule in a buffer ordinarily used for biochemical purposes at an appropriate concentration and the solution is injected onto a microplate.

After the two molecules are brought into contact with each other, and preferably after a process is applied to wash excess labeled target molecule not bound to the molecule immobilized on the solid phase with the same buffer or the like, a molecule that interacts with the antigen molecule immobilized on the solid phase can be identified by using a marketed ELISA reader or the like to measure or analyze fluorescence generated from the labeled molecule remaining on the solid phase.

As a method for simultaneously performing multiple analyses by the aforementioned method, for example, a method may be employed in which plural different labeled target molecules are immobilized in each well of the microplate.

### (3-5) Fluorescence depolarization method

The fluorescence depolarization (Perran, J., et al., J. Phys. Rad., 1, 390-401 (1926)) is a method utilizing the fact that a fluorescent molecule excited by polarized fluorescence emits fluorescence in the same polarized plane during the excited state when it maintains a steady state, whilst when the excited molecule is in rotational Brownian motion or the like, the emitted fluorescence gives a polarized plane different from that of the excitation light. The molecular motion is effected by a size of the molecule. When a fluorescent molecule is a polymer, the molecule can hardly be in motion during the excited state and the emitted light maintains polarization, whereas emitted light of a low molecular fluorescent molecule is depolarized because of a fast velocity of the motion. Thus, information concerning kinetics and existing state of a molecule can be obtained based on a ratio of intensities of fluorescence emitted by the fluorescent molecule excited by a plane polarized light measured for the original plane and a plane perpendicular thereto. According to the method, behavior of a target molecule that interacts with a molecule labeled with fluorescence can be traced without influence of impurities, if any. This is because change of polarization degree is measured only when the molecule labeled with fluorescence and the target molecule interact with each other.

As apparatuses for performing the above method, for example, BECON (Panyera) and so forth are commercially available, and this method can be performed by using these apparatuses.

When measurement or analysis of an interaction between a protein and a molecule is performed by using this method, both of the C-terminus labeled protein and the target molecule are required to be supplied as a solution. The target molecule need not be labeled. Molecule which have a molecular weight significantly smaller than that of the C-terminus labeled protein of which interaction is to be analyzed are not suitable for this method, because such molecules will not effect on the Brownian motion of the C-terminus labeled protein.

As methods for bringing the target molecule into contact with the C-terminus labeled protein in the aforementioned method, any methods may be employed so long as they can achieve contact of the two molecules in a sufficient degree for their interaction. Preferably, the contact is preferably attained by a method of introducing a solution, in which the C-terminus labeled protein is dissolved in a buffer ordinarily used for biochemical purposes or the like at an appropriate concentration, into wells for measurement of a commercially available fluorescence depolarization apparatus, and further introducing into the wells a solution in which the target molecule is dissolved at an appropriate concentration in the same buffer.

In the interaction between the C-terminus labeled protein and the target molecule analyzed by the above method, specificity may sometimes be expected not so high as that of the antigen/antibody method. Accordingly, it may be effective to evaluate degree of interaction as numerical values to detect an optimum combination. As a marker of the degree of interaction, for example, a value of minimum target concentration may be used which gives the maximum fluorescence polarization for a constant concentration of the C-terminus labeled protein.

As methods for simultaneously performing multiple analyses by the aforementioned method, for example, applicable methods include a method of introducing each of plural different C-terminus labeled proteins into each well for measurement of the aforementioned fluorescence depolarization measurement apparatus and then introducing a solution of a particular target molecule to each well, or a method of introducing a particular C-terminus labeled protein into wells and then introducing each of solutions of plural different kinds of target molecules to each well.

### (3-6) Fluorescence correlation spectroscopy

The fluorescence correlation spectroscopy (FCS, Eigen, M., et al., Proc. Natl. Acad. Sci. USA, 91, 5740-5747 (1994)) is a method in which flowing velocity, diffusion coefficient, volume shrinkage and the like of particles are measured under a confocal laser microscope or the like. In the present invention, a molecule with interaction can be determined by measuring change of translational Brownian motion which is caused on the basis of an interaction between the C-terminus labeled protein and the target molecule.

Specifically, a sample particle is excited by an excitation light, and a part of sample solution volume radiates fluorescence. This radiated light is measured to obtain a photon ratio. This value changes in connection with a number of particles existing in a space volume observed at a given time. The various parameters mentioned above can be calculated from changes of the signal by using autocorrelation functions. Apparatuses for performing the FCS are also commercially available from Carl Zeiss and so forth, and analysis according to the above method can be performed by using these apparatuses.

When measurement or analysis of an interaction between a protein and a molecule is performed by using this method, both of the C-terminus labeled protein and the target molecule are required to be supplied as a solution. The target molecule need not be labeled. Molecule which have a molecular weight significantly smaller than that of the C-terminus labeled protein of which interaction is to be analyzed are not suitable for this method, because such molecules will not effect on the Brownian motion of the C-terminus labeled protein

As methods for bringing the target molecule into contact with the C-terminus labeled protein in the aforementioned method, any methods may be employed so long as they can achieve contact of the two molecules in a sufficient degree for their interaction. Preferably, the contact is preferably attained by a method of introducing a solution, in which the C-terminus labeled protein is dissolved in a buffer ordinarily used for biochemical purposes or the like at an appropriate concentration, into wells for measurement of a commercially available FCS apparatus, and further introducing into the wells a solution in which the target molecule is dissolved at an appropriate concentration in the same buffer.

As methods for simultaneously performing multiple analyses by the aforementioned method, for example, applicable methods include a method of introducing each of plural different C-terminus labeled proteins into each well for measurement of the aforementioned FCS apparatus and then introducing a solution of a particular target molecule to each well, or a method of introducing a particular C-terminus labeled protein into wells and then introducing each of solutions of plural different kinds of target molecules to each well.

### (4) Method for identifying molecule that causes interaction

As for a molecule which is determined and observed to have an interaction with the C-terminus labeled protein by any of the methods mentioned in the above (3), its primary structure can be analyzed by an appropriate method that is known per se when the primary structure of the molecule is unknown. Specifically, when the target molecule which is observed to have an interaction is a protein, its amino acid sequence can be analyzed by an amino acid analysis apparatus or the like to specify the primary structure. Further, when the target molecule is a nucleic acid, its nucleotide sequence can be determined by using an automatic DNA sequencer or the like based on a nucleotide sequencing method.

### (5) Apparatus for immobilizing C-terminus labeled protein on solid phase

In order to perform a method for immobilizing the C-terminus labeled protein on a solid phase via its labeled moiety described in the above (1-3), it is also possible to construct an apparatus by combining suitable known means. Each means of the apparatus is known, per se, and each of the operations in the means such as holding of a base plate, addition of a solution of the C-terminus labeled protein, and washing may be performed by methods known, per se. By combining these means, a full automatic or semi-automatic apparatus for immobilizing a C-terminus labeled protein on a solid phase can be constructed.

### (6) Apparatus for measurement of interaction between protein and molecule

In order to perform measurement of an interaction between a protein and a molecule described in the above (3), an apparatus can also be constructed by combining known suitable means. Each means of the apparatus is known, per se, and each of the operations in the means such as holding of a base plate, addition of the target molecule, washing, and detection of signal can be performed by methods known, per se. By combining these means, a full automatic or semi-automatic apparatus for measurement of an interaction between a protein and a molecule can be constructed.

### Examples

The present invention will be further specifically explained with reference to the following examples. However, the following examples should be construed only as aids for better understanding of the present invention, and the scope of the present invention is not limited by the following examples.

### Example 1: Analysis of increased efficiency for labeling of C-terminus of a protein using a template of which stop codon is deleted

### (1) Construction of DNA for transcription and preparation of mRNA

A DNA fragment, in which a DNA sequence encoding a β-lactamase was ligated in the downstream of a region containing a DNA sequence recognizable by the RNA polymerase of *Escherichia coli* virus T7 with a high transcription efficiency (T7 promoter sequence) and a DNA sequence readily recognizable by eukaryotic ribosome during translation (Kozak consensus sequence), was constructed as follows.

A single-stranded DNA containing the T7 promoter sequence (Rosenberg, A.H., et al., Gene, 56, 125-135 (1987)) and the Kozak consensus sequence (SEQ ID NO: 1) was chemically synthesized, and used together with a DNA primer (SEQ ID NO: 2) and a DNA/RNA primer (SEQ ID NO: 3) to perform polymerase chain reaction (PCR). Separately, DNA encoding a β-lactamase was amplified using a β-lactamase gene by PCR with a DNA/RNA primer (SEQ ID NO: 4) and a DNA primer (SEQ ID NO: 5). According to the RRR method (Nishigaki, K., et al., Chem. Lett., 131-132 (1995)), ribonuclease A (Sigma) was added to each PCR reaction mixture and the mixture was allowed to react at 60°C for 30 minutes for cleavage of phosphodiester bonds at the 3' end of the RNA of the DNA/RNA primers (SEQ ID NOS: 3 and 4) to form a cohesive end. The products were extracted with phenol, and then the primers and digested DNA fragments were removed by using Primer Remover (Edge Biosystems). The resulting products were further subjected to ethanol precipitation. The precipitates were dried and then dissolved in a buffer for T4 DNA ligase and added with T4 polynucleotide kinase (NEB). The mixture was allowed to react at 45°C for 30 minutes. Then, the temperature of the reaction mixture was gradually lowered to 16°C over 30 minutes and then added with T4 DNA ligase (NEB) to ligate the above two DNA regions. A part of the reaction mixture was collected and amplified again by PCR using DNA primers (SEQ ID NOS: 2 and 5) to obtain a template for transcription. For PCR, AmpliTaq Gold DNA Polymerase (Perkin-Elmer) was used.

The same procedure as described above was carried out except that a DNA primer (SEQ ID NO: 6) in which the stop codon of the β-lactamase was removed was used instead of the aforementioned DNA primer (SEQ ID NO: 5) to prepare a template for transcription containing a DNA encoding the β-lactamase in which the stop codon was removed.

The DNA encoding the β -lactamase having the stop codon and the DNA encoding the β -lactamase in which the stop codon was removed prepared as described above were transcribed into RNA by using an RNA synthesis kit (Ribomax Large Scale RNA Production System, Promega). In order to increase synthesis efficiency, a Cap analogue (RNA Capping Analogue, Gibco BRL) was used to modify the 5' end of mRNA. Ethanol precipitation was performed by using Primer Remover (Edge Biosystems) to remove the cap analogue and excessive substrate (NTP).

### (2) Preparation of fluorescence labeling agent

Puromycin (SIGMA) was dissolved in 3 ml of dry pyridine and dehydrated by evaporation under reduced pressure. This procedure was repeated three times. The residue was added with 5 ml of 4% tetrazole solution in acetonitrile and FluoreDite (6-N-carboxy-di-O-pivaloyl-fluorescein-hexyl-O-(2-cyanoethyl)-(N,N'-diisopropyl)-phosphoamidite, Japan Perceptive) and stirred at room temperature. The reaction was monitored by silica gel thin layer chromatography (TLC, developing solvent: chloroform:methanol = 9:1). The reaction was usually completed in 2 hours. After the reaction was completed, the solvent was removed under reduced pressure, and the residue was added with 2 ml of a solution in which 0.1 M iodine was dissolved in tetrahydrofuran/pyridine/water = 80:40:2 to oxidize the resulting phosphite triester with stirring at room temperature. After 90 minutes, the solvent was removed under reduced pressure, and the residue was extracted with chloroform. The extract was dried in the presence of anhydrous magnesium sulfate, and the solvent was removed under reduced pressure. The residue was applied to silica gel TLC and elution was performed with chloroform/methanol = 90:10. When the elution was performed with chloroform/methanol = 90:10 in the silica gel TLC, fluorescenylpuromycin (Fluorpur) having the protective group was eluted in a fraction at Rf of 0.26.

The eluted Fluorpur having the protective group was added to 1 ml of a mixed solution of concentrated aqueous ammonia/ethanol = 2:1 to remove the β-cyanoethyl group. After the reaction, 7 mg of Fluorpur was obtained. The synthesized product was verified as Fluorpur by peaks at 272 nm (originated from puromycin moiety) and 494 nm (originated from fluorescein moiety) in ultraviolet and visible absorption spectra of a solution at pH 9 and a molecular ion of [M+H]⁺ appeared at m/z 1010 in MALDI/TOF mass spectrometry.

### (3) Preparation of C-terminus labeled protein

The mRNA prepared in the above (1) was allowed to react in a rabbit reticulocyte cell free translation system (Rabbit Reticulocyte Lysate Systems, Nucleease Treated, Promega) and a wheat germ cell free translation system (Wheat Germ Extract, Promega), each added with Fluorpur prepared in the above (2) at a final concentration of 16 µM, at an optimum reaction temperature for each system (rabbit reticulocyte cell free translation system: 30°C, wheat germ cell free translation system: 25°C) for 60 minutes.

### (4) Evaluation of the labeling of protein

Each of the reaction mixtures of the cell translation systems used in the above (3) was subjected to SDS polyacrylamide electrophoresis (SDS-PAGE) according to the method of Schagger et al. (Schagger, H. and von Jagows, G., Anal. Biochem., 166, 368-379 (1987)) at a constant voltage of 20 V for 90 minutes, and then fluorescence intensity of the gel was measured by a fluorescence imaging analyzer (FluorImager 595, Molecular Dynamics).

The β-lactamase protein most efficiently labeled with fluorescence was that prepared in the wheat germ cell free translation system by using the β -lactamase mRNA whose stop codon was deleted. The labeling efficiency was 10 times or higher than the protein obtained by using the RNA transcribed from the DNA encoding the β -lactamase having the stop codon in the same system.

Also in the rabbit reticulocyte cell free translation system, use of the RNA transcribed from the DNA encoding the β -lactamase in which the stop codon was deleted gave labeling efficiency 3 to 4 times higher than the protein obtained by using the RNA having the stop codon.

### Example 2: Analysis of interaction between partial B domain of C-terminus labeled protein A and human IgG using fluorescence depolarization method

### (1) Construction of DNA fragment encoding B domain and construction of its mRNA

A DNA fragment, in which a DNA sequence encoding B domain of protein A was ligated in downstream of a region having a DNA sequence recognizable by the RNA polymerase of *Escherichia coli* virus T7 with high transcription efficiency (T7 promoter sequence), a sequence readily recognizable by eukaryotic ribosome during translation (Kozak consensus sequence), and a sequence readily recognizable by prokaryotic ribosome (Shine-Dalgarno sequence), was constructed as follows.

DNAs for a region having T7 promoter sequence, Kozak consensus sequence and Shine-Dalgarno sequence and a region having a sequence coding for the B domain were separately prepared. A single-stranded DNA containing the T7 promoter sequence (Rosenberg, A.H., et al., Gene, 56, 125-135 (1987)), the Kozak consensus sequence (SEQ ID NO: 1) and the Shine-Dalgarno sequence was chemically synthesized (SEQ ID NO: 7) and used together with a DNA primer (SEQ ID NO: 8) and a primer coding for a part of the B domain (SEQ ID NO: 9) to perform polymerase chain reaction (PCR). As the DNA synthetase, TaKaRaEx Taq Polymerase (Takara Shuzo) was used.

Separately, a DNA region encoding the B domain was amplified using a pRIT2T plasmid (New England Biolab) as a template which carried a protein A gene by performing polymerase chain reaction with an antisense primer of SEQ ID NO: 8 (SEQ ID NO: 10) and DNA primer (SEQ ID NO: 11). These two PCR products were ligated according to the overlap extension method (Horton R.M., et al., Gene, 77, 61-68 (1989)) and amplified by PCR using two DNA primers (SEQ ID NOS: 8 and 11) to prepare a DNA fragment encoding the B domain. As the DNA synthetase, TaKaRaEx Taq Polymerase (Takara Shuzo) was used for each PCR.

Using an RNA synthesis kit (Ribomax Large Scale RNA Production System, Promega), the DNA prepared by the method described above was transcribed into mRNA by adding 10 µg of the DNA to each 100 µl of the reaction mixture. In order to increase transcription efficiency, a Cap analogue (RNA Capping Analogue, Gibco BRL) was added at a final concentration of 7.2 mM to modify the 5' side of mRNA. Ethanol precipitation was performed by using a primer removing agent (Primer Remover, Edge Biosystems) to remove the cap analogue and excessive NTP (nucleotide triphosphate).

### (2) Preparation of fluorescence labeling agent, fluorescein-puromycin (Fluorpur)

Puromycin (SIGMA, 26 mg, 48 µmol) was dissolved in 3 ml of dry pyridine and dehydrated by evaporation under reduced pressure. This procedure was repeated three times. The residue was added with 5 ml of 4% tetrazole solution in acetonitrile and FluoreDite (6-N-carboxy-di-O-pivaloyl-fluorescein-hexyl-O-(2-cyanoethyl)-(N,N'-diisopropyl)-phosphoamidite, Japan Perceptive) and stirred at room temperature. The reaction was monitored by silica gel thin layer chromatography (TLC, developing solvent: chloroform:methanol = 9:1). The reaction was usually completed in 2 hours. After the reaction, the solvent was removed under reduced pressure, and the residue was added with 2 ml of a solution in which 0.1 M iodine was dissolved in tetrahydrofuran/pyridine/water = 80:40:2 to oxidize the resulting phosphite triester with stirring at room temperature.

After 1 hour and 30 minutes, the solvent was removed under reduced pressure, and the residue was extracted with chloroform. The extract was dried in the presence of anhydrous magnesium sulfate, and the solvent was removed. The residue was applied to silica gel column chromatography (Merck) and elution was performed with chloroform/methanol = 90:10. Fluorpur having the protective group was eluted in a fraction at Rf of 0.26 in the silica gel TLC (developing solvent, chloroform/methanol = 9:1). Then, the protective group was removed.

Fluorpur having the protective group was added to 1 ml of a mixed solution of concentrated aqueous ammonia/ethanol = 2:1 for cleavage of the β -cyanoethyl group to obtain 7 mg of Fluorpur. The synthesized product was verified as Fluorpur based on based on the peaks at 272 nm (originated from puromycin moiety) and 494 nm (originated from fluorescein moiety) in ultraviolet and visible absorption spectra of a solution at pH 9 and a molecular ion appeared at m/z 1010 in MALDI/TOF mass spectrometry.

### (3) Preparation of C-terminus labeled protein

The mRNA prepared was allowed to react at 37°C for 60 minutes in a translation system utilizing a cell free translation kit, *E. coli* S30 extract (Promega) by adding fluorenylpuromycin (Fluorpur) at a final concentration of 16 µM. In order to remove unreacted Fluorpur, the reaction mixture was eluted from a PD-10 column (BIO-RAD) equilibrated with 25 ml of TBS buffer (10 mM Tris-HCl, 150 mM NaCl, pH 8.0). The labeled B domain was eluted in the top fraction of 1.6 ml. For verification by electrophoresis, the fraction was further concentrated to about 130 µl by centrifugation using Centricon 3 (Amicon).

### (4) Evaluation of the labeling of the protein

The reaction mixture of the cell translation system obtained in the above (3) was subjected to SDS polyacrylamide electrophresis (SDS-PAGE) according to the method of Schagger et al. (Schagger, H. and von Jagows, G., Anal. Biochem., 166, 368-379 (1987)) at a constant voltage of 20 V for 90 minutes, and then fluorescence intensity of the gel was measured by a fluorescence imaging analyzer (FluorImager 595, Molecular Dynamics).

Furthermore, concentration of the labeled B domain protein was measured based on fluorescein intensities which were obtained from absorbance at 494 nm in a solution of pH 9 measured by a fluorospectrophotometer (RF-502, Shimadzu).

### (5) Analysis of interaction between B domain and human IgG using fluorescence depolarization measurement apparatus

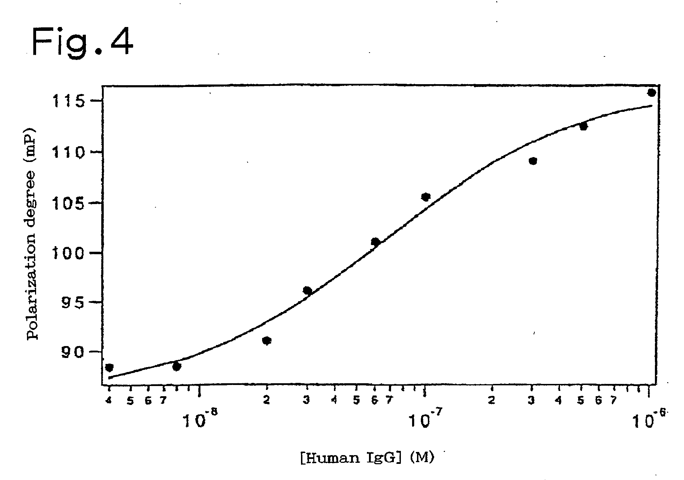

Solutions of labeled B domain protein at concentrations of about 0.1 to 1 nM were prepared and added with samples of human IgG (SIGMA) at concentrations of 0.01 nM to 10 µM obtained by 10-fold dilution. After the reaction mixtures were left stand for 30 minute at room temperature, each polarization degree of the sample was measured by a fluorescence depolarization measurement apparatus (BEACON 2000, PanVera). The results of the polarization degree for each human IgG concentration are shown in Fig. 4. A dissociation constant was calculated based on these measured values. As a result, KD was found to be 6.4 × 10⁻⁸. The results were well correlated with known data.

### Example 3: Immobilization of biotinylated protein on streptavidin membrane

### (1) Construction of DNA fragment encoding GFPuv4 and construction of its mRNA

Polymerase chain reaction (PCR) was performed with a primer DNA (SEQ ID NO: 8) and a primer encoding a part of GFPuv4 (SEQ ID NO: 12) by using a single-stranded DNA as a template which contained the T7 promoter sequence, Kozak consensus sequence and Shine-Dalgarno sequence. As the DNA synthetase, KOD Polymerase (Toyobo) was used.

Separately, a DNA region encoding GFPuv4 was amplified by performing polymerase chain reaction with an antisense primer of SEQ ID NO: 12 (SEQ ID NO: 13) and a primer for 3' end of GFP gene (SEQ ID NO: 14) by using DNA coding for GFPuv4 (Ito, Y., et al., Biochem. Biophys. Res. Commun., 264 (2), 556-60 (1999)) as a template.

These two PCR products were ligated according to the overlap extension method (Horton R.M., et al., Gene, 77, 61-68 (1989)) and amplified by PCR with two DNA primers (SEQ ID NOS: 8 and 14) to prepare a DNA chain in which the DNA encoding GFPuv4 was ligated downstream of the T7 promoter. As the DNA synthetase, KOD Polymerase (Toyobo) was used for each PCR.

By using an RNA synthesis kit (Ribomax Large Scale RNA Production System, Promega), the DNA prepared by the method described above was transcribed into mRNA by adding 10 µg of the DNA to each 100 µl of the reaction mixture. In order to increase transcription efficiency, a Cap analogue (RNA Capping Analogue, Gibco BRL) was added at a final concentration of 7.2 mM to modify the 5' side of mRNA. Ethanol precipitation was performed by using a primer removing agent (Primer Remover, Edge Biosystems) to remove the cap analogue and excessive NTP (nucleotide triphosphate).

### (2) Preparation of biotin labeling agent, biotin-puromycin (Biotin-Puro)

Puromycin (SIGMA) of which amino group and 5'-hydroxyl group were protected with trifluoroacetyl group and dimethoxytrityl group, respectively, was immobilized via the 3'-hydroxyl group on a solid phase carrier (NovasYn TG Amino Resin LL, Nova Biochem), and biotin (Biotin TEG Phosphoramidide, Glen Research) or PEG spacer (Spacer 18, Glen Research) was bound to the resulting product on a nucleic acid synthesizer (ABI 3498, Perkin-Elmer) according to the phosphoamidite method. Then, the protective groups were removed.

### (3) Labeling of a protein

The resulting mRNA ( 2 µg) was allowed to react at 26°C for 60 minutes in a translation system utilizing a cell free translation kit, Wheat Germ Extract (Promega), by adding 2 µl of 500 µM biotin-puromycin (Biotin-puro) or 2 µl of 500 µM polyethylene glycol-puromycin (PEG-puro). In order to remove unreacted Biotin-puro or PEG-puro, each reaction mixture was eluted from a PD-10 column (BIO-RAD) equilibrated with 25 ml of TBS buffer (10 mM Tris-HCl, 150 mM NaCl, pH 8.0).

### (4) Evaluation of the labeling of the protein

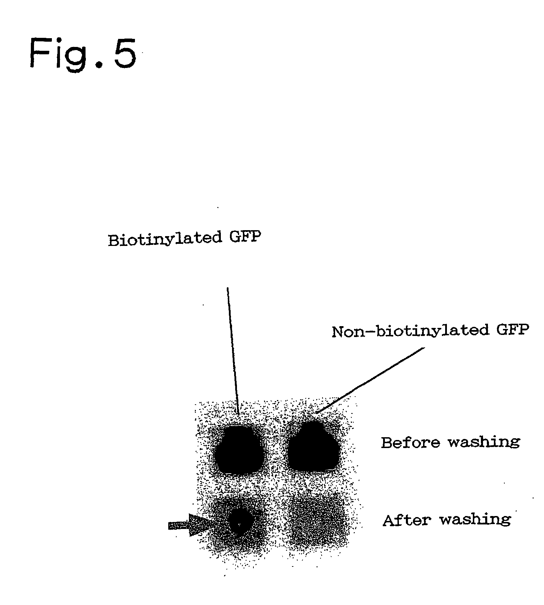

For verification of the protein labeled with biotin-puromycin (Biotin-puro), each 15 µl of the above samples was spotted on SAM2 Biotin Capture Membrane (Promega) and left stand for 10 minutes. Then, the membrane was washed with TBS buffer (10 mM Tris-HCl, 150 mM NaCl, pH 8.0). Fluorescence intensity on the membrane was imaged by using FluoroImager FX (Bio-Rad) before and after the washing with the TBS buffer. The results are shown in Fig. 5. Biotin-puro and PEG-puro gave almost same intensities before the washing with TBS buffer. After the washing, GFPuv4 labeled with PEG-puro was removed by the washing, whilst GFPuv4 labeled with Biotin-puro was immobilized on the membrane via biotin.

### Example 4: Analysis of interaction between B domain of biotinylated protein A and human IgG using fluorescence image analyzer

### (1) Construction of DNA fragment coding for protein A, B domain and GFPuv4 and construction of its mRNA

Polymerase chain reaction (PCR) was performed with a primer DNA (SEQ ID NO: 8) and a primer containing a nucleotide sequence encoding a part of B domain of protein A and a linker sequence (nucleotide sequence encoding Gly-Gly-Gly-Gly-Ser) (SEQ ID NO: 15) by using a single-stranded DNA containing the T7 promoter sequence, Kozak consensus sequence and Shine-Dalgarno sequence (SEQ ID NO: 7) as a template. As the DNA synthetase, KOD Polymerase (Toyobo) was used.

Separately, a DNA region encoding GFPuv4 (Ito, Y., et al., Biochem. Biophys. Res. Commun., 264 (2), 556-60 (1999)) ligated with the linker sequence at its 5' end was amplified by performing polymerase chain reaction by using a DNA encoding GFPuv4 as a template with a primer containing an antisense sequence of the linker sequence of SEQ ID NO: 15 and a nucleotide sequence encoding a part of GFPuv4 (SEQ ID NO: 16) and 3' primer for a DNA encoding GFP (SEQ ID NO: 14).

These two PCR products were ligated according to the overlap extension method (Horton R.M., et al., Gene, 77, 61-68 (1989)) and amplified by PCR using two DNA primers (SEQ ID NOS: 8 and 14) to prepare a DNA chain in which, downstream of the T7 promoter, the DNA encoding B domain of protein A was ligated to the DNA encoding GFPuv4 via a linker sequence. As the DNA synthetase, KOD Polymerase (Toyobo) was used for each PCR.

Using an RNA synthesis kit (Ribomax Large Scale RNA Production System, Promega), the DNA prepared by the method described above was transcribed into mRNA by adding 10 µg of the DNA to each 100 µl of the reaction mixture. In order to increase transcription efficiency, a Cap analogue (RNA Capping Analogue, Gibco BRL) was added at a final concentration of 7.2 mM to modify the 5' side of mRNA. Ethanol precipitation was performed by using a primer removing agent (Primer Remover, Edge Biosystems) to remove the cap analogue and excessive NTP (nucleotide triphosphate).

### (2) Preparation of biotin labeling agent, biotin-puromycin (Biotin-Puro)

Puromycin (SIGMA) of which amino group and 5'-hydroxyl group were protected with trifluoroacetyl group and dimethoxytrityl group, respectively, was immobilized via the 3'-hydroxyl group on a solid phase carrier (NovasYn TG amino resin LL, Nova Biochem), and a PEG spacer (Spacer Phosphoramidide 18, Glen Research) and biotin (Biotin TEG Phosphoramidide, Glen Research) were successively bound to the resulting product on a nucleic acid synthesizer (ABI 3498, Perkin-Elmer) according to the phosphoamidite method. Then, the protective groups were removed.

### (3) Labeling of protein

The mRNA prepared (2 µg) was allowed to react at 26°C for 60 minutes in a translation system utilizing a cell free translation kit, Wheat Germ Extract (Promega) by adding 2 µl of 500 µM biotin-puromycin (Biotin-puro). In order to remove unreacted Biotin-puro, gel filtration was performed in a Bio-spin column (BIO-RAD).

### (4) Immobilization of C-terminus biotin labeled B domain-GFPuv4 binding protein

The C-terminus biotin labeled B domain-GFPuv4 binding protein (Biotin-Domain B-GFPuv4) prepared in the above (3) was added in an amount of 50 µ/l to each well of SAM2 Biotin Capture Plate (Promega) and left stand for 15 minutes, and then each well was washed with TBS buffer (10 mM Tris-HCl, 150 mM. NaCl, pH 8.0). Then, in order to coat streptavidin adhered to a plate that was not bound with biotin, 100 µl of 1 mM biotin/PBS was added to each well. After the washing, each well was washed 5 times with 200 µl of TBS buffer. The wells were then blocked with 1% BSA (SIGMA)/PBS. Wells in which Biotin-Domain B-GFPuv4 was not immobilized was similarly blocked as a control.

### (5) Analysis of interaction between B domain immobilized on solid phase and human IgG by fluorescence imaging analyzer

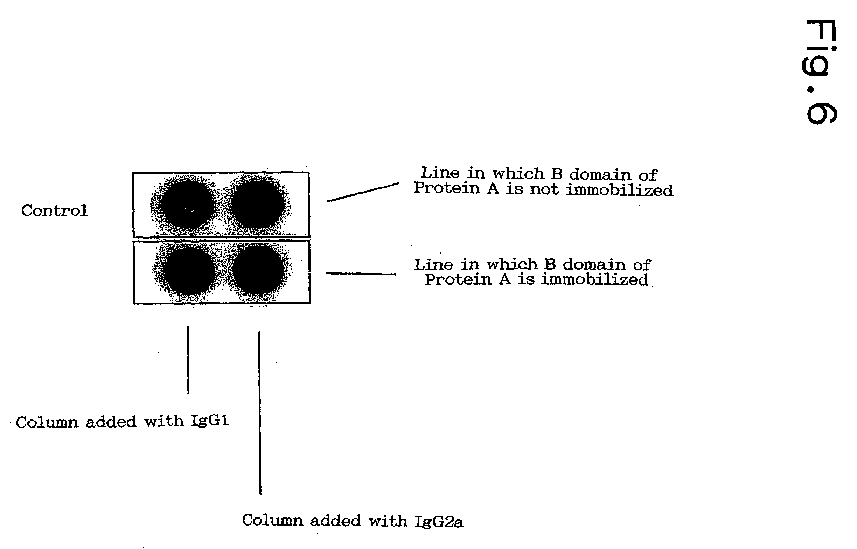

Each well of SAM2 Biotin Capture Plate in which Biotin-Domain B-GFPuv4 was immobilized or not immobilized prepared in the above (4) was added with Monoclonal Anti-Human IgG I Biotin Conjugate with high affinity for B domain (Mouse IgG2a isotype, SIGMA) or Biotin-Mouse Monoclonal Anti-Human IgA I with low affinity for B domain (mouse IgG1 isotype, SIGMA), which were diluted with PBS buffer 50,000 times, and then the mixture was left stand for 1 hour. Each well was washed three times with TBS buffer, added with ExtraAvidin-Alkaline Phosphatase (SIGMA), left stand for 10 minutes and then washed three times with TBS buffer. Each well was further added with Attphos Substrate (Amersham Pharmacia Biotech) and allowed to react at room temperature for 20 minutes. The wells were imaged by using a fluorescence imaging analyzer (FluorImager FX, Bio-Rad) and the results are shown in Fig. 6.

In the well in which Biotin-Domain B-GFPuv4 was not immobilized, only background fluorescence was observed. Whilst, as for the wells in which Biotin-Domain B-GFPuv4 was immobilized, fluorescence in the wells introduced with Mouse IgG2a isotype with high affinity for B domain was stronger than fluorescence in the wells introduced with mouse IgG1 isotype with low affinity for B domain, which indicates that the degree of the interaction was successfully measured and analyzed.

### Industrial Applicability

According to the present invention, a means is provided for quickening analysis of functions of proteins encoded by nucleotide sequences of genes whose data have been rapidly accumulated recently, for example, analyses of protein-protein interaction, protein-nucleic acid interaction, protein-low molecular compound interaction and the like. According to the present invention, a useful means is provided for a method of rapidly analyzing a large number of data (realization of higher throughput), which is indispensable means for functional analysis of proteins.

## Claims

1. A method for analyzing an interaction between a protein and a molecule, which comprises the following steps:
(1) a step of bringing a C-terminus labeled protein into contact with a target molecule, and
(2) a step of detecting a change of a signal which is caused an interaction between the C-terminus labeled protein and the target molecule among signals generated by the C-terminus labeled protein or the target molecule.

2. The method according to claim 1, wherein the target molecule is a protein, a nucleic acid, a sugar chain, or a low molecular compound.

3. The method according to claim 1 or 2, wherein the target molecule is a labeled molecule which is labeled with a labeling substance.

4. The method according to claim 3, wherein the labeling substance is fluorescence labeling agent.

5. The method according to any one of claims 1 to 4, wherein the C-terminus labeled protein is prepared by expressing a nucleic acid comprising a coding region for the protein in a transcription and/or translation system to allow protein synthesis in the presence of a labeling agent which comprises a labeling moiety comprising a labeling substance and an acceptor moiety comprising a compound having an ability to bind to the C-terminus of a protein.

6. The method according to claim 5, wherein the acceptor moiety comprises a compound containing a chemical skeleton of puromycin, 3'-N-aminoacylpuromycin aminonucleoside, or 3'-N-aminoacyladenosine aminonucleoside, or an analogue of said compound.

7. The method according to claim 5 or claim 6, wherein the labeling agent comprises a compound in which the labeling moiety binds to the acceptor moiety via a spacer.

8. The method according to any one of claims 1 to 7, wherein either of the C-terminus labeled protein or the target molecule is bound to a solid phase.

9. The method according to claim 8, wherein the C-terminus labeled protein is bound to the solid phase at the labeled moiety or at a moiety other than the labeled moiety which constitutes said protein.

10. The method according to claim 9, wherein the labeled moiety constituting the C-terminus labeled protein comprises a molecule having an ability to specifically bind to a particular polypeptide, wherein said molecule is bound to the solid phase via a linkage with said particular polypeptide bound to a surface of the solid phase.

11. The method according to claim 10, wherein a combination of the particular polypeptide and the molecule having an ability to specifically bind to the particular polypeptide is any one of combination selected from the group consisting of biotin binding protein/biotin, maltose binding protein/maltose, G protein/guanine nucleotide, polyhistidine peptide/metal ion, glutathione-S-transferase/glutathione, DNA binding protein/DNA, antibody/antigen molecule, calmodulin/calmodulin binding peptide, adenosine triphosphate (ATP) binding protein/ATP and estradiol receptor protein/estradiol.

12. The method according to any one of claims 1 to 11, wherein the change of the signal is measured by one or more methods selected from the group consisting of surface plasmon resonance method, evanescent field imaging method, fluorescence imaging analysis method, enzyme linked immunosorbent assay, fluorescence depolarization method, and fluorescence correlation spectroscopy.

13. The method according to any one of claims 1, 2 and 5 to 11, wherein the C-terminus labeled protein is bound to the solid phase via the labeled moiety constituting the protein, wherein the target molecule is an unlabeled molecule or a molecule labeled with a labeling substance, and wherein the change of the signal is measured by one or more of methods selected from the group consisting of surface plasmon resonance method, evanescent field imaging method, fluorescence imaging analysis method, and enzyme linked immunosorbent assay.

14. The method according to claim 13, wherein the labeled molecule is the target molecule labeled with a fluorescence labeling agent.

15. The method according to any one of claims 1, 2 and 5 to 11, wherein the C-terminus labeled protein is bound to the solid phase via a moiety other than the labeled moiety which constitutes the protein, wherein the target molecule is an unlabeled molecule or a molecule labeled with a labeling substance, and wherein the change of the signal is measured by one or more of methods selected from the group consisting of surface plasmon resonance method, evanescent field imaging method, fluorescence imaging analysis method and enzyme linked immunosorbent assay.

16. The method according to claim 15, wherein the labeled molecule is the target molecule labeled with a fluorescence labeling agent.

17. The method according to any one of claims 1, 2 and 5 to 11, wherein the target molecule is bound to the solid phase, and wherein the change of the signal generated from the labeled moiety of the C-terminus labeled protein is measured by one or more of methods selected from the group consisting of surface plasmon resonance method, evanescent field imaging method, fluorescence imaging analysis method, and enzyme linked immunosorbent assay.

18. The method according to any one of claims 1, 2 and 5 to 11, wherein the C-terminus labeled protein and the target molecule are not bound to the solid phase but exist in a phase of a solution, wherein the target molecule is an unlabeled molecule or a molecule labeled with a labeling substance, and wherein the change of the signal is measured by fluorescence depolarization method and/or fluorescence correlation spectroscopy.

19. A labeling agent for a protein comprising a labeling moiety and an acceptor moiety, wherein the labeling moiety is a molecule having an ability to specifically bind to a particular polypeptide, and the acceptor moiety is a compound having an ability to bind to a C-terminus of a protein.

20. The agent according to claim 19, wherein the labeling moiety is bound to the acceptor moiety via a spacer.

21. The agent according to claim 19 or claim 20, wherein the spacer is either polyethylene or polyethylene glycol.

22. The agent according to any one of claims 19 to 21, wherein the labeling moiety comprises one or more molecules selected from the group consisting of biotin, maltose, guanine nucleotide, metal ion, glutathione, protein binding DNA, antigen molecule, calmodulin binding peptide, ATP, and estradiol.

23. The agent according to any one of claims 19 to 22, wherein the acceptor moiety comprises a compound containing a chemical skeleton of puromycin, 3'-N-aminoacylpuromycin aminonucleoside or 3'-N-aminoacyladenosine aminonucleoside, or an analogue of said compound.

24. An immobilized protein **characterized in that** a C-terminus labeled protein is bound to a solid phase via a labeled moiety which constitutes the protein.

25. The immobilized protein according to claim 24, wherein the C-terminus labeled protein is prepared by expressing a nucleic acid containing a coding region for the protein in a transcription and/or translation system to allow protein synthesis in the presence of a labeling agent comprising a labeling moiety containing a labeling substance and an acceptor moiety containing a compound having an ability to bind to a C-terminus of a protein.

26. The immobilized protein according to claim 24 or claim 25, wherein the labeling moiety of the labeling agent is covalently bound to the acceptor moiety via a spacer.

27. The immobilized protein according to any one of claims 24 to 26, wherein the labeling moiety is a molecule having an ability to specifically bind to a particular polypeptide and wherein the molecule is bound to the particular polypeptide which is bound to the solid phase.

28. The immobilized protein according to any one of claims 24 to 27, wherein the acceptor moiety comprises a compound having a chemical skeleton of puromycin, 3'-N-aminoacylpuromycin aminonucleoside or 3'-N-aminoacyladenosine aminonucleoside, or an analogue of said compound.

29. The immobilized protein according to any one of claims 24 to 28, wherein a combination of the particular molecule constituting the labeling moiety and the molecule having an ability to specifically bind to the molecule is a combination selected from the group consisting of biotin binding protein/biotin, maltose binding protein/maltose, G protein/guanine nucleotide, polyhistidine peptide/metal ion, glutathione-S-transferase/glutathione, DNA binding protein/DNA, antibody/antigen molecule, calmodulin/calmodulin binding peptide, ATP binding protein/ATP, and estradiol receptor protein/estradiol.

30. A protein chip comprising an assembly of the immobilized proteins according to any one of claims 24 to 29.

31. An apparatus for producing the protein chip according to claim 30, which comprises a means for holding a base plate whose surface is bound with an adapter protein, a means for introducing a C-terminus labeled protein into the base plate, and a means for washing the base plate.

32. An apparatus for simultaneous analyses of multiple test samples by the method according to any one of claims 10, 11, 13 and 14, which comprises a means for holding a chip, a means for adhering a target molecule to the chip, a means for washing the chip, and a means for measuring a signal from the C-terminus labeled protein and detecting a change of a signal on the basis of an interaction between the C-terminus labeled protein and the target molecule.

33. A method for identifying a molecule which interacts with a protein or identifying a protein which interacts with a molecule, which comprises the steps of:
(1) a step of preparing a C-terminus labeled protein by labeling C-terminus of the protein,
(2) a step of bringing the C-terminus labeled protein into contact with the target molecule, and
(3) a step of judging that the C-terminus labeled protein and the target molecule have interaction when a change of a signal from the C-terminus labeled protein or the target molecule is detected wherein said change is caused by the contact of the C-terminus labeled protein and the target molecule.

34. The method according to claim 33, which further comprises the following step:
(4) a step of identifying the protein and the target molecule which are judged to have interaction.

35. A method for identifying a molecule which interacts with a particular protein or identifying a protein which interacts with a particular molecule, which comprises the following steps:
(1) a step of bringing a C-terminus labeled protein comprising the protein into contact with the target molecule, and
(2) a step of judging that the C-terminus labeled protein and the target molecule have interaction when a change of a signal from the C-terminus labeled protein or the target molecule is detected wherein said change is caused by the contact of the C-terminus labeled protein and the target molecule.

36. The method of claim 35, which further comprises the following step:
(3) a step of identifying the protein and the target molecule which are judged to have interaction.

37. A method for screening a molecule which interacts with a protein or screening a protein which interacts with a molecule, which comprises the following steps:
(1) a step of bringing a C-terminus labeled protein comprising the protein into contact with the target molecule, and
(2) a step of judging that the C-terminus labeled protein and the target molecule have interaction when a change of a signal from the C-terminus labeled protein or the target molecule is detected wherein said change is caused by the contact of the C-terminus labeled protein and the target molecule.

38. A molecule having interaction with said protein or a protein having interaction with said molecule screened by the method according to claim 37.

39. A C-terminus labeled protein for use in the method according to any one of claims 33 to 36.

40. A C-terminus labeled protein for use in the method according to claim 37.
